(19) Europäisches Patentamt
European Patent Office
Office européen des brevets

(11) **EP 2 653 158 B1**

(12) **EUROPEAN PATENT SPECIFICATION**

(45) Date of publication and mention
of the grant of the patent:
**05.07.2017 Bulletin 2017/27**

(51) Int Cl.:
*A61K 31/155* *(2006.01)*    *A61K 45/00* *(2006.01)*
*A61P 3/04* *(2006.01)*    *A61P 3/10* *(2006.01)*
*A61P 9/12* *(2006.01)*    *A61P 25/00* *(2006.01)*
*A61P 25/08* *(2006.01)*    *A61P 25/18* *(2006.01)*
*A61P 25/26* *(2006.01)*    *A61P 25/28* *(2006.01)*
*A61P 31/04* *(2006.01)*

(21) Application number: **11848995.4**

(22) Date of filing: **12.12.2011**

(86) International application number:
**PCT/JP2011/079266**

(87) International publication number:
**WO 2012/081713 (21.06.2012 Gazette 2012/25)**

(54) **THERAPEUTIC AGENT FOR BLOOD-BRAIN BARRIER DISRUPTION SYNDROME**

THERAPEUTIKUM GEGEN STÖRUNGEN DER BLUT-HIRN-SCHRANKE

AGENT THÉRAPEUTIQUE POUR UN SYNDROME DE RUPTURE DE LA BARRIÈRE HÉMATO-ENCÉPHALIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **13.12.2010 JP 2010277027**

(43) Date of publication of application:
**23.10.2013 Bulletin 2013/43**

(73) Proprietor: **Fukuoka University
Fukuoka-shi, Fukuoka 814-0180 (JP)**

(72) Inventors:
• **TAKATA, Fuyuko
Fukuoka-shi, Fukuoka 814-0180 (JP)**
• **DOHGU, Shinya
Fukuoka-shi, Fukuoka 814-0180 (JP)**
• **KATAOKA, Yasufumi
Fukuoka-shi, Fukuoka 814-0180 (JP)**
• **MATSUMOTO, JunIchi
Fukuoka-shi, Fukuoka 814-0180 (JP)**
• **KANESHIMA, Syuji
Fukuoka-shi, Fukuoka 814-0180 (JP)**

(74) Representative: **Duckworth, Timothy John
J A Kemp
14 South Square
Gray's Inn
London WC1R 5JJ (GB)**

(56) References cited:
**WO-A2-2007/124308**

• **TAKATA FUYUKO ET AL: "Metformin induces up-regulation of blood-brain barrier functions by activating AMP-activated protein kinase in rat brain microvascular endothelial c",** BIOCHEMICAL AND BIOPHYSICAL RESEARCH COMMUNICATIONS, ACADEMIC PRESS INC. ORLANDO, FL, US, vol. 433, no. 4, 21 March 2013 (2013-03-21), pages 586-590, XP028582709, ISSN: 0006-291X, DOI: 10.1016/J.BBRC.2013.03.036
• YOKOYAMA, Y. ET AL.: 'Regulation of glyceraldehyde 3-phosphate dehydrogenase expression by metformin in HepG2 cells' BIOLOGICAL & PHARMACEUTICAL BULLETIN vol. 32, no. 7, 2009, pages 1160 - 1165, XP055108915
• TAKAYUKI MATSUMOTO: 'A Therapeutic Target for Endothelium-derived Hyperpolarizing Factor Signaling in Diabetic Vascular Complication' JOURNAL OF THE PHARMACEUTICAL SOCIETY OF JAPAN vol. 130, no. 6, 01 June 2010, pages 777 - 784, XP055108916
• DOHGU, S. ET AL.: 'Cyclosporin A induces hyperpermeability of the blood-brain barrier by inhibiting autocrine adrenomedullin-mediated up-regulation of endothelial barrier function' EUROPEAN JOURNAL OF PHARMACOLOGY vol. 644, no. 1-3, 2010, pages 5 - 9, XP027243308

EP 2 653 158 B1

- SU, E.J. ET AL.: 'Activation of PDGF-CC by tissue plasminogen activator impairs blood-brain barrier integrity during ischemic stroke' NATURE MEDICINE vol. 14, no. 7, 2008, NEW YORK, NY, UNITED STATES, pages 731 - 737, XP055108917
- SHARMA, R.V. ET AL.: 'Metformin attenuates agonist-stimulated calcium transients in vascular smooth muscle cells' CLINICAL AND EXPERIMENTAL HYPERTENSION vol. 17, no. 6, 1995, pages 913 - 929, XP008168313
- LEYBAERT, L. ET AL.: 'Inositol-trisphosphate-dependent intercellular calcium signaling in and between astrocytes and endothelial cells' GLIA vol. 24, no. 4, 1998, pages 398 - 407, XP055108918
- CHARLES, M.A. ET AL.: 'Effect of weight change and metformin on fibrinolysis and the von Willebrand factor in obese nondiabetic subjects: the BIGPR01 Study.' BIGUANIDES AND THE PREVENTION OF THE RISK OF OBESITY, DIABETES CARE vol. 21, no. 11, 1998, pages 1967 - 1972, XP055108920

## Description

TECHNICAL FIELD

The present invention relates to a therapeutic agent for blood-brain barrier dysfunction syndrome. More specifically, the present invention relates to a therapeutic agent for blood-brain barrier dysfunction syndrome comprising a biguanides agent which is metformin or buformin or a pharmaceutically acceptable salt thereof as an active ingredient for treating or preventing various diseases by enhancing blood-brain barrier functions.

Background Art

The blood-brain barrier (BBB) is composed principally of cerebrovascular endothelial cells and separates circulating blood from the brain parenchyma. This barrier restricts the transport of substances such as drugs into the brain. In addition, the blood-brain barrier that is composed of cerebrovascular endothelial cells together with brain pericytes and glia cells maintains higher brain functions by forming a "cerebral neurovascular unit" that integrates network functions together with brain neurons. These blood-brain barrier dysfunctions are involved in the formation of various pathological conditions of brain dysfunction (Non Patent Literature 1).

The pathological conditions caused by blood-brain barrier dysfunction such as a decline in blood-brain barrier functions include, for example, (i) the increased amount of drugs transferred into the brain, (ii) cerebral edema attributed to the brain infiltration of serum albumin, (iii) encephalopathy attributed to leukocyte infiltration into the brain, and (iv) the defective cerebral clearance of biologically active peptides such as amyloid beta protein (Aβ). These pathological conditions cause (1) the occurrence of adverse reactions (side effects) of drugs and the onset or worsening of diseases attributed to blood-brain barrier dysfunction (hereinafter, these diseases are also collectively referred to as blood-brain barrier dysfunction syndrome), such as (2) cerebral infarction or cerebral trauma, (3) neurodegenerative disease in the brain, and (4) Alzheimer's disease.

Imatinib, a therapeutic agent for chronic myeloid leukemia or the like, is known to suppress blood-brain barrier permeability, thereby suppressing cerebral infarction (Non Patent Literature 2). Also, pranlukast, a therapeutic agent for bronchial asthma or the like, is known to serve as a vascular hyperpermeability-suppressing agent that acts on the lumens of capillary vessels and vascular endothelial cells constituting the lumen structure to prevent plasma components, blood cells, etc., in the capillary vessels from being leaked into tissue (Patent Literature 1).

However, any pharmaceuticals agent for treating blood-brain barrier dysfunction syndrome by enhancing blood-brain barrier functions has not yet been in actual use.

Citation List

Patent Literature

Patent Literature 1: Japanese Patent Laid-Open No. 2009-221217
Patent Literature 2: PCT Publication No. WO 2007/124308

Non Patent Literature

Non Patent Literature 1: Neuron, 2008, 57, 178-201
Non Patent Literature 2: Nature Medicine, 2008, July, 14(7), 731-737
Non Patent Literature 3: Nicolas W., Florence M., Sylvie C., Pierre-Olivier C., "The blood-brain barrier in brain hoeostasis and neurological diseases", Biochimica et Biophysica Acta, 2009, 1778, p.842-857
Non Patent Literature 4: Nishioku T., Yamauchi A., Takata F., Watanabe T., Furusho K., Shuto H., Dohgu S., Kataoka Y., "Disruption of the blood-brain barrier in collagen-induced arthritic mice", Neuroscience Letters. 2010, October 4, 482(3), p.208-211
Non Patent Literature 5: Yamauchi A., Dohgu S., Takata F., Watanabe T., Nishioku T., Matsumoto J., Ohkubo Y., Shuto H., Kataoka Y., "Partial hepatectomy aggravates cyclosporin A-induced neurotoxicity by lowering the function of the blood-brain barrier in mice", Life Sciences, 2011, March 14, 88(11-12), p.529-534
Non Patent Literature 6: Dohgu S., Sumi N., Nishioku T., Takata F., Watanabe T., Naito M., Shuto H., Yamauchi A., Kataoka Y., "Cyclosporin A induces hyperpermeability of the blood-brain barrier by inhibiting autocrine adrenomedullin-mediated up-regulation of endothelial barrier function", European Journal of Pharmacology, 2010, October 10, 644(1-3), p.5-9
Non Patent Literature 7: Dohgu S., Nishioku T., Sumi N., Takata F., Nakagawa S., Naito M., Tsuruo T., Yamauchi A., Shuto H., Kataoka Y, "Adverse effect of cyclosporin A on barrier functions of cerebral microvascular endothelial cells after hypoxia-reoxygenation damage in vitro", Cellular and Molecular Neurobiology, 2007, November,27(7), p. 889-899
Non Patent Literature 8: Takata F., Dohgu S., Yamauchi A., Sumi N., Nakagawa S., Naito M., Tsuruo T., Shuto H., Kataoka Y, "Inhibition of transforming growth factor-beta production in brain pericytes contributes to cyclosporin A-induced dysfunction of the blood-brain barrier", Cellular and Molecular Neurobiology, 2007, May, 27(3), p.317-328
Non Patent Literature 9: Yamauchi A., Shuto H., Dohgu S., Nakano Y, Egawa T., Kataoka Y, "Cyclosporin A aggravates electroshock-induced convulsions in mice with a transient middle cerebral artery occlusion", Cellular and Molecular Neurobiology, 2005, August, 25(5), p.923-928 Non Patent Literature 10: Dohgu S., Yamauchi A., Nakagawa S., Takata F., Kai M., Egawa T., Naito M., Tsuruo T., Sawada Y, Niwa M., Kataoka Y., "Nitric oxide mediates cyclosporine-induced impairment of the blood-brain barrier in cocultures of mouse brain endothelial cells and rat astrocytes", European Journal of Pharmacology, 2004, November 28, 505(1-3), p.51-59 Non Patent Literature 11: Yamauchi A., Oishi R., Kataoka Y, "Tacrolimus-induced neurotoxicity and nephrotoxicity is ameliorated by administration in the dark phase in rats", Cell Mol Neurobiol, 2004, October, 24(5), p.695-704

Non Patent Literature 12: Fujisaki Y, Yamauchi A., Dohgu S., Sunada K., Yamaguchi C., Oishi R., Kataoka Y, "Cyclosporine A-increased nitric oxide production in the rat dorsal hippocampus mediates convulsions", Life Sciences, 2002, December 20, 72(4-5), p.549-556 Non Patent Literature 13: Tominaga K., Kai M., Yamauchi A., Dohgu S., Toda K., Oishi R., Kataoka Y, "Subchronic treatment with cyclosporin A decreases the binding properties of the GABAA receptor in ovariectomized rats", Life Sciences, 2002, December 20, 72(4-5), p.425-430 Non Patent Literature 14: Kanoski SE, Zhang Y, Zheng W., Davidson TL, "The effects of a high-energy diet on hippocampal function and blood-brain barrier integrity in the rat", Journal of Alzheimer's Disease, 2010, January, 21(1), p.207-219

Non Patent Literature 15: Ueno M, Sakamoto H, Tomimoto H, Akiguchi I, Onodera M, Huang CL, Kanenishi K., "Blood-brain barrier is impaired in the hippocampus of young adult spontaneously hypertensive rats.", Acta Neuropathologica, 2004, June, 107(6), p.532-538 Non Patent Literature 16: Honda M., Nakagawa S., Hayashi K., Kitagawa N., Tsutsumi K., Nagata I., Niwa M., "Adrenomedullin improves the blood-brain barrier function through the expression of claudin-5", Cellular and Molecular Neurobiology, 2006, 26(2), p.109-118 Non Patent Literature 17: Jin R., Yang G., Li G., "Molecular insights and therapeutic targets for blood-brain barrier disruption in ischemic stroke: critical role of matrix metalloproteinases and tissue-type plasminogen activator", Neurobiol of Disease, 2010, Jun, 38(3), p.376-385 Non Patent Literature 18: Abu Fanne R., Nassar T., Yarovoi S., Rayan A., Lamensdorf I., Karakoveski M., Vadim P., Jammal M., Cines D. B., Higazi A. A., "Blood-brain barrier permeability and tPA-mediated neurotoxicity", Neuropharmacology, 2010, Jun, 58(7), p.972-980 Non Patent Literature 19: Yokoyama Y., Kubota M., Iguchi K., Usui S., Kiho T., Hirano K., "Regulation of glyceraldehyde 3-phosphate dehydrogenase expression by metformin in HepG2 cells", Biological & Pharmaceutical Bulletin, 2009, 32(7), p.1160-1165

Non Patent Literature 20: Matsumoto T., "A therapeutic target for endothelium-derived hyperpolarizing factor signaling in diabetic vascular complication", Journal of the Pharmaceutical Society of Japan, 2010, 130(6), p.777-784

Non Patent Literature 21: Su E., Fredriksson L., Geyer M., Folestad E., Cale J., Andrae J., Gao Y., Pietras K., Mann K., Yepes M., Strickland D., Betsholtz C., Eriksson U., Lawrence D., "Activation of PDGF-CC by tissue plasminogen activator impairs blood-brain barrier integrity during ischemic stroke", Nature Medicine, 2008, 14(7), p.731-737

Non Patent Literature 22: Sharma R., Bhalla R., "Metformin attenuates agonist-stimulated calcium transients in vascular smooth muscle cells", Clinical and Experimental Hypertension, 1995, 17(6), p.913-929

Non Patent Literature 23: Leybaert L., Paemeleire K., Strahonja A., Sanderson M., "Inositol-triphosphate-dependent intercellular calcium signaling in and between astrocytes and endothelial cells", Glia, 1998, 24(4), p.398-407

Non Patent Literature 24: Charles M., Morange P., Eschwège E., André P., Vague P., Juhan-Vague I., "Effect of weight change and metformin on fibrinolysis and the von Willebrand factor in obese nondiabetic subjects: the BIGPRO1 study. Biguanides and the prevention of the risk of obesity", Diabetes Care, 1998, 21(11), p.1967-1972

WO 2007/124308 (Patent Literature 2) discloses methods and compositions for modulating blood-neural barrier (BNB) for the treatment of CNS conditions such as edema, and for increased drug delivery efficacy across the BNB.

Dohgu et al. (Non Patent Literature 6) teaches that cyclosporin A, a potent immunosuppressant, may inhibit the adenylyl cyclase/cyclic AMP/PKA signaling pathway activated by adrenomedullin, leading to impairment of brain endothelial barrier function.

Yokoyama et al. (Non Patent Literature 19) teaches that signal transducers, adenylate cyclase (AC), protein kinase A (PKA), and AMP-activated protein kinase (AMPK), may be involved in the signaling pathway triggered by metformin and cAMP-response element (CRE)-binding protein may be one of the transcription factors for the glyceraldehyde 3-phosphate dehydrogenase (GAPD) gene down-regulated by metformin. A decrease in the expression of GAPD is suggested to be one of the causes of lactic acidosis.

Matsumoto (Non Patent Literature 20) teaches that chronic treatment with metformin, eicosapentaenoic acid, or thromboxane synthase inhibitor can reduce contracting factor (EDCF) signaling and normalize hyperpolarizing factor (EDHF) signaling in mesenteric arteries from type 2 diabetic rats.

Su et al. (Non Patent Literature 21) provides data which are said to demonstrate that platelet-derived growth factor (PDGF) signaling regulates blood-brain barrier permeability and to suggest potential new strategies for stroke treatment.

Sharma *et al.* (Non Patent Literature 22) teaches that metformin may mediate its vascular effects by attenuating agonist-stimulated intracellular calcium concentration.

Leybaert *et al.* (Non Patent Literature 23) teaches that an increase of inositol triphosphate ($IP_3$) in a single cell is sufficient to initiate an intercellular $Ca^{2+}$ wave that is propagated by the diffusion of $IP_3$ to neighboring cells and that can be communicated between astrocytes and endothelial cells in co-culture. By contrast, $Ca^{2+}$ diffusion via gap junctions does not appear to be sufficient to propagate an intercellular $Ca^{2+}$ wave. They suggest that intercellular $Ca^{2+}$ waves may play a role in astrocyte-endothelial interactions at the blood-brain barrier.

Charles *et al.* (Non Patent Literature 24) teaches that in nondiabetic men (n = 151) and women (n = 306) aged between 34 and 65 years with a central fat distribution and a mean BMI of 32.5 kg/m2, weight loss was the main factor associated with the decrease in plasminogen activator inhibitor 1 (PAI-1), in accordance with the recent demonstration of production of PAI-1 by adipocytes. Metformin had a significant effect on two factors, tissue-type plasminogen activator (tPA) antigen and von Willebrand factor (vWF), mainly secreted by the endothelial cells, which is said to suggest an effect of the drug on the production or the metabolism of these two hemostatic proteins.

Summary of Invention

Technical Problem

An object of the present invention is to provide a pharmaceutical composition for treating or preventing various diseases by enhancing blood-brain barrier functions.

Solution to Problem

As a result of conducting diligent studies, the present inventor has completed the present invention by finding that a medicament comprising a biguanides agent which is metformin or buformin or a pharmaceutically acceptable salt thereof as an active ingredient or a pharmaceutical composition containing the medicament enhances blood-brain barrier functions including tight junction between cerebrovascular endothelial cells, transcellular transport, etc., thereby suppressing the leakage of albumin present in brain capillary or a tight junction marker fluorescein sodium into brain tissue.

In this context, the term "enhancement" used in the present specification and claims means enhancement of a function decreased congenitally or after birth as well as enhancement of a normal function and further means enhancement of a function by "reconstruction" that improves the function decreased congenitally or after birth. This term also means "suppression" of a decline in function.

In addition, the term also means "promotion" of enhancement of a function through an external or internal factor.

Thus, the enhancement of blood-brain barrier functions including tight junction, transcellular transport, etc. shall be interpreted not only as being the enhancement of the blood-brain barrier functions but also as including the reconstruction of the blood-brain barrier functions, the suppression of a decline in the blood-brain barrier functions, and the promotion of the enhancement of the blood-brain barrier functions, the reconstruction thereof, and the suppression of the decline.

Specifically, the present application provides a therapeutic agent for blood-brain barrier dysfunction syndrome which is a medicament or a pharmaceutical composition comprising a biguanides agent which is metformin or buformin or a pharmaceutically acceptable salt thereof as an active ingredient for enhancing a blood-brain barrier function.

More specifically, the present application provides a therapeutic agent for blood-brain barrier dysfunction syndrome according to the present invention for preventing worsening or recurrence of cerebral infarction. The present application also provides a therapeutic agent for blood-brain barrier dysfunction syndrome according to the present invention for preventing, suppressing, or ameliorating cerebral edema associated with cerebral infarction. The present application further provides a therapeutic agent for blood-brain barrier dysfunction syndrome according to the present invention for preventing, suppressing, or ameliorating encephalopathy associated with sepsis. The present application further provides a therapeutic agent for blood-brain barrier dysfunction syndrome according to the present invention for preventing, suppressing, or ameliorating leukocyte infiltration into the brain in multiple sclerosis. The present application further provides a therapeutic agent for blood-brain barrier dysfunction syndrome according to the present invention for preventing, suppressing, or ameliorating accumulation of amyloid beta protein ($A\beta$) in the brain in Alzheimer's disease.

In addition, the present application provides a therapeutic agent for blood-brain barrier dysfunction syndrome according to the present invention for preventing or suppressing drug transport into the brain, thereby preventing, suppressing, or ameliorating the adverse reaction that affects an animal or human central nervous system.

The present invention relates to the following items, though some items are already described above:

(Item 1) A medicament for use in treating blood-brain barrier dysfunction syndrome, comprising a biguanides agent which is metformin or buformin or a pharmaceutically acceptable salt thereof as an active ingredient.

(Item 2) The medicament for use according to item 1, wherein the blood-brain barrier dysfunction syndrome is derived from a disease selected from the group consisting of diabetes mellitus, obesity, hypertension, cerebral infarction, cerebral trauma, sepsis, multiple sclerosis, Alzheimer's disease, and combination thereof, or attributed to a substance inducing the central adverse reactions.

(Item 3) The medicament for use according to item 1, wherein the medicament is used in combination with a thrombolytic drug.

(Item 4) The medicament for use according to item 1, wherein the medicament prevents or suppresses transport of an immunosuppressive or thrombolytic drug into the brain.

(Item 5) The medicament for use according to item 4, wherein the medicament prevents or suppresses said drug transport into the brain, thereby preventing, suppressing, or ameliorating an adverse reaction that affects the animal or human central nervous system, preferably wherein said adverse reaction is selected from tremor, convulsion, leukoencephalopathy, headache, sleepiness, disturbance of consciousness, abnormal behavior, delirium, hallucination, delusion, epilepsy, cerebral infarction, cerebral hemorrhage and combination thereof.

(Item 6) The medicament for use according to item 4 or 5, wherein said drug is an immunosuppressive drug.

(Item 7) The medicament for use according to item 6, wherein the immunosuppressive drug is cyclosporine A (CsA).

(Item 8) The medicament for use according to item 4 or 5, wherein said drug is a thrombolytic drug.

(Item 9) The medicament for use according to item 8, wherein the thrombolytic drug is tissue plasminogen activator (t-PA).

(Item 10) The medicament for use according to any one of items 1 to 9, wherein said biguanides agent or the pharmaceutically acceptable salt thereof is metformin hydrochloride or buformin hydrochloride.

(Item 11) The medicament for use according to item 10 which said biguanides agent or the pharmaceutically acceptable salt thereof is metformin hydrochloride.

(Item 12) A medicament for use in treating blood-brain barrier dysfunction syndrome in combination with cyclosporin A (CsA) or tissue plasminogen activator (t-PA), comprising a biguanides agent or a pharmaceutically acceptable salt thereof as an active ingredient, and wherein said biguanides agent or the pharmaceutically acceptable salt thereof is metformin hydrochloride.

(Item 13) The medicament for use according to any one of items 1 to 12, wherein a dose of the active ingredient is 125 to 3000 mg/day/person.

The present invention further relates to the following items, though some items are already described above:

(Item 1a) A therapeutic agent for blood-brain barrier dysfunction syndrome comprising a biguanides agent which is metformin or buformin or a pharmaceutically acceptable salt thereof as an active ingredient for preventing, suppressing, or ameliorating cerebral edema associated with cerebral infarction or cerebral trauma.

(Item 2a) A therapeutic agent for blood-brain barrier dysfunction syndrome comprising a biguanides agent which is metformin or buformin or a pharmaceutically acceptable salt thereof as an active ingredient for preventing, suppressing, or ameliorating encephalopathy associated with sepsis.

(Item 3a) A therapeutic agent for blood-brain barrier dysfunction syndrome comprising a biguanides agent which is metformin or buformin or a pharmaceutically acceptable salt thereof as an active ingredient for preventing, suppressing, or ameliorating leukocyte infiltration into the brain in multiple sclerosis.

(Item 4a) A therapeutic agent for blood-brain barrier dysfunction syndrome comprising a biguanides agent which is metformin or buformin or a pharmaceutically acceptable salt thereof as an active ingredient for preventing, suppressing, or ameliorating accumulation of amyloid beta protein (Aβ) in the brain in Alzheimer's disease.

(Item 5a) A therapeutic agent for blood-brain barrier dysfunction syndrome comprising a biguanides agent which is metformin or buformin or a pharmaceutically acceptable salt thereof as an active ingredient for preventing or suppressing worsening or recurrence of cerebral infarction.

(Item 6a) A therapeutic agent for blood-brain barrier dysfunction syndrome comprising a biguanides agent which is metformin or buformin or a pharmaceutically acceptable salt thereof as an active ingredient for enhancing a blood-brain barrier function.

(Item 7a) The therapeutic agent for blood-brain barrier dysfunction syndrome according to item 6a, wherein said enhancement of the blood-brain barrier function is prevention, suppression, or promotion of the suppression of a decline in the blood-brain barrier function.

(Item 8a) The therapeutic agent for blood-brain barrier dysfunction syndrome according to item 7a, wherein said decline in the blood-brain barrier function is attributed to a disease selected from the group consisting of diabetes mellitus, obesity, hypertension, and combination thereof.

(Item 9a) The therapeutic agent for blood-brain barrier dysfunction syndrome according to any one of items 1a to 8a, wherein said biguanides agent or the pharmaceutically acceptable salt thereof is metformin hydrochloride or buformin hydrochloride.

(Item 10a) The therapeutic agent for blood-brain barrier dysfunction syndrome according to any one of items 1a to 9a, wherein a dose of the active ingredient is 125 to 3000 mg/day/person.

Advantageous Effects of Invention

The therapeutic agent for blood-brain barrier dysfunction syndrome according to the present invention is effective for enhancing blood-brain barrier functions including tight junction between cerebrovascular endothelial cells, transcellular transport, etc., thereby preventing, suppressing, or ameliorating a disease or a symptom attributed to blood-brain barrier dysfunction.

Brief Description of Drawings

Fig. 1 is a schematic diagram of an *in vitro* BBB model test.
Fig. 2 is a graph showing that the medicament of the present invention suppressed the permeability of albumin.
Fig. 3 is a graph showing that the medicament of the present invention suppressed the permeability of fluorescein sodium.
Fig. 4 is a graph showing that the medicament of the present invention suppressed the hyperpermeability of fluorescein sodium induced by CsA in the blood-brain barrier.
Fig. 5 is a graph showing that the medicament of the present invention suppressed the hyperpermeability of fluorescein sodium induced by t-PA in the blood-brain barrier.
Fig. 6 is a graph showing that the medicament of the present invention suppressed the hyperpermeability of albumin induced by t-PA in the blood-brain barrier.
Fig. 7 is a photograph of the brain of a cerebral infarction mouse model.
Fig. 8 is a graph showing that the medicament of the present invention suppressed the hyperpermeability of fluorescein sodium induced by t-PA in the blood-brain barrier of a cerebral infarction mouse model.
Fig. 9 is a graph showing that the medicament of the present invention suppressed the hyperpermeability of albumin induced by t-PA in the blood-brain barrier of a cerebral infarction mouse model.

Description of Embodiments

The therapeutic agent for blood-brain barrier dysfunction syndrome according to the present invention enhances blood-brain barrier functions including tight junction between cerebrovascular endothelial cells, transcellular transport, etc. As a result, the medicament or the pharmaceutical composition of the present invention prevents, suppresses, or ameliorates the pathological conditions caused by blood-brain barrier dysfunction, thereby contributing to the prevention, suppression, or amelioration of a disease or a symptom attributed to blood-brain barrier dysfunction, such as the diseases described above.

Examples of an active ingredient in the therapeutic agent for blood-brain barrier dysfunction syndrome according to the present invention include biguanides agents such as metformin and buformin, and pharmaceutically acceptable salts thereof.

When the therapeutic agent for blood-brain barrier dysfunction syndrome according to the present invention comprises a pharmaceutically acceptable salt of a biguanides agent as an active ingredient, the salt is not limited by a type. Any inorganic or organic acid salt can be used. Examples of an inorganic acid for the inorganic acid salt include hydrochloric acid, sulfuric acid, nitric acid, phosphoric acid, and boric acid. Examples of a salt for the organic acid salt include formic acid, acetic acid, propionic acid, lactic acid, benzoic acid, oxalic acid, succinic acid, fumaric acid, maleic acid, malic acid, tartaric acid, citric acid, methanesulfonic acid, and benzenesulfonic acid. Preferable examples of the salt include hydrochloride. In this context, metformin hydrochloride and buformin hydrochloride are commercially available as hypoglycemic agents.

The biguanides agent of the present invention or the pharmaceutically acceptable salt thereof is not limited by a production method and can be produced by a method known in the art.

Alternatively, the biguanides agent of the present invention or the pharmaceutically acceptable salt thereof can be made into a preparation according to a routine method, either alone or in combination with a pharmaceutically acceptable pharmaceutical carrier known in the art.

The therapeutic agent for blood-brain barrier dysfunction syndrome according to the present invention is not limited by an administration method. Examples thereof include oral administration, administration through intravenous injection, administration through intramuscular injection, administration through intraperitoneal injection, administration through hypodermic or intradermal injection, intrarectal administration, transmucosal administration, and administration via the respiratory tract. Preferable examples of the administration method include oral administration.

The therapeutic agent for blood-brain barrier dysfunction syndrome according to the present invention is not limited by its dosage form. Examples of the dosage form for oral administration include tablets, granules, capsules, powders, solutions, suspensions, and syrups. Examples of the dosage form for parenteral administration include injections, intravenous drops, suppositories, and percutaneously absorbable formulations.

The pharmaceutical composition in any of the dosage forms described above can be produced using a pharmaceutically acceptable pharmaceutical carrier known in the art. For example, an excipient, a binder, a disintegrant, a lubricant, a stabilizer, a coloring agent, and a corrigent can be used.

Examples of the excipient include acrylic acid starch, gum arabic, lactose, corn starch, saccharose, glucose, sorbitol, crystalline cellulose, silicon dioxide calcium silicate, and magnesium silicate. Examples of the binder include polyvinyl alcohol, calcium citrate, carboxyvinyl polymer, carboxymethylethylcellulose, polyvinyl ether, methylcellulose, gum arabic, tragacanth, gelatin, shellac, hydroxypropylcellulose, hydroxypropylmethylcellulose, ethylcellulose, dextrin, sodium polyphosphate, and pectin. Examples of the disintegrant include: disintegrants called super disintegrants such as crospovidone, croscarmellose sodium, and carmellose calcium; hydroxypropylcellulose; carboxymethyl starch sodium; and corn starch. Examples of the lubricant include magnesium stearate, talc, polyethylene glycol, aluminum stearate, lactose, magnesium carbonate, carmellose calcium, carmellose sodium, and hydrogenated plant oil. Examples of the stabilizer include sodium edetate, sodium sulfite, butylhydroxyanisole, and butylhydroxytoluene. Examples of the coloring agent include: caramel; edible dyes or edible lake dyes such as Food Yellow No. 5, Food Red No. 2, and Food Blue No. 2; and iron red. Examples of the corrigent include hydrochloric acid, orange oil, fennel, cacao powder, menthol, aromatic acid, peppermint oil, and cinnamon bark oil. The tablets or the granules may be sugar-coated, gelatin-coated, or coated in any other manner, if necessary.

The medicament or the pharmaceutical composition according to the present invention can be administered to a human at a dose of 125 to 3000 mg/ day /person in terms of metformin hydrochloride. The dose may be, for example, 250 mg/ day / person, 500 mg/ day / person, 750 mg/ day / person, 850 mg/ day / person, 1500 mg/ day / person, 1700 mg/ day / person, 2250 mg/ day / person, or 2550 mg/ day / person.

As mentioned above, blood-brain barrier dysfunction causes the onset of various pathological conditions or the exacerbation or recurrence of the pathological conditions and is predisposed to, for example, the onset, worsening, or recurrence of a disease related to the blood-brain barrier dysfunction. For example, sepsis is thought to cause a decline in blood-brain barrier functions, which in turn causes transport of neurotoxins from blood into the brain, resulting in encephalopathy (Non Patent Literature 3). Also, the increased expression of adhesion molecules on cerebrovascular endothelial cells constituting the blood-brain barrier is thought to allow leukocytes to easily permeate the blood-brain barrier, resulting in the progression of multiple sclerosis (Non Patent Literatures 3 and 4). In addition, a blood-brain barrier dysfunction is considered to allow amyloid beta protein (Aβ) to accumulate in the brain, resulting in the progression of Alzheimer's disease (Non Patent Literature 3).

Thus, the agent for enhancing a blood-brain barrier function according to the present invention is useful in the prevention, suppression, or amelioration of the diseases or the symptoms attributed to blood-brain barrier dysfunction.

For example, the therapeutic agent for blood-brain barrier dysfunction syndrome according to the present invention can be used for preventing or suppressing worsening or recurrence of cerebral infarction. The therapeutic agent for blood-brain barrier dysfunction syndrome according to the present invention can also be used for preventing, suppressing, or ameliorating cerebral edema associated with cerebral infarction. The therapeutic agent for blood-brain barrier dysfunction syndrome according to the present invention can also be used for preventing, suppressing, or ameliorating cerebral edema associated with cerebral trauma. The therapeutic agent for blood-brain barrier dysfunction syndrome according to the present invention can also be used for preventing, suppressing, or ameliorating encephalopathy associated with sepsis. The therapeutic agent for blood-brain barrier dysfunction syndrome according to the present invention can also be used for preventing, suppressing, or ameliorating leukocyte infiltration into the brain in multiple sclerosis. The therapeutic agent for blood-brain barrier dysfunction syndrome according to the present invention can also be used for preventing, suppressing, or ameliorating accumulation of amyloid beta protein (Aβ) in the brain in Alzheimer's disease.

In an alternative embodiment of the present invention, the therapeutic agent for blood-brain barrier dysfunction syndrome according to the present invention can be used for preventing or suppressing transport of an immunosuppressive or thrombolytic drug into the brain. The drug may be a drug that possesses the unwanted effects on an animal or human central nervous system. Alternatively, the drug may be a drug that possesses the unwanted effects on an animal or human central nervous system. In such a case, the medicament or the pharmaceutical composition of the present invention may be used even for suppressing transport of the drug into the brain in an amount more than expected.

In addition, the medicament or the pharmaceutical composition of the present invention can be used for preventing or suppressing the drug transport into the brain, thereby preventing, suppressing, or ameliorating an adverse symptom that affects the animal or human central nervous system.

For example, an immunosuppressive drug (cyclosporine, tacrolimus, etc.)-induced blood-brain barrier dysfunctions and following blood-brain barrier hyperpermeability are known to be responsible for adverse reactions such as tremor, convulsion, leukoencephalopathy, and headache that result from central adverse reactions (Non Patent Literatures 5 to 12). The present invention can suppress such a decline in blood-brain barrier functions or enhance or reconstruct the blood-brain barrier functions, thereby preventing, suppressing, or ameliorating the adverse symptoms.

The present invention can also prevent, suppress, or ameliorate a thrombolytic drug (tissue plasminogen activator, etc.)-induced blood-brain barrier dysfunctions. The present invention can also prevent, suppress, or ameliorate hemorrhagic infarction associated with the blood-brain barrier dysfunctions. The present invention can also be used for expanding a therapeutic window during the application of the thrombolytic drug to the treatment of cerebral infarction. Therefore, the present invention can be used as a therapeutic agent for cerebral infarction in combination with the thrombolytic drug.

In addition, the present invention can prevent, suppress, or ameliorate sleepiness caused by antihistamine drugs, disturbance of consciousness, abnormal behavior, delirium, hallucination, delusion, convulsion, etc., caused by Tamiflu, convulsion or epilepsy caused by interferon, infliximab, antidepressants, or new quinolone antimicrobial agents, and leukoencephalopathy caused by carmofur, tegafur, or fluorouracil.

In an alternative embodiment of the present invention, the therapeutic agent for blood-brain barrier dysfunction syndrome according to the present invention or the medicament or the pharmaceutical composition of the present invention can be used for preventing, suppressing, or ameliorating a decline in blood-brain barrier functions.

For example, diabetes mellitus, obesity, or hypertension is known to possibly cause a decline in blood-brain barrier functions (Non Patent Literatures 14 and 15). Therefore, the therapeutic agent for blood-brain barrier dysfunction syndrome according to the present invention can be used for preventing, suppressing, or ameliorating a decline in blood-brain barrier functions induced by diabetes mellitus, obesity, hypertension, or the like. The prevention, suppression, or amelioration of the blood-brain barrier dysfunctions leads to prevention of worsening or recurrence of cerebral infarction, prevention or suppression of drug transfer to the brain, etc.

Meanwhile, such a decline in blood-brain barrier functions may cause onset or worsening of diabetes mellitus and/or obesity. Therefore, the therapeutic agent for blood-brain barrier dysfunction syndrome according to the present invention can also be used for preventing, suppressing, or ameliorating the diseases attributed to a decline in blood-brain barrier functions.

In addition, the biguanides agent or the salt thereof, or the substance or the composition comprising it as an active ingredient can also be used as a reagent for animal tests for the effects or uses described above. The animal can be selected from, for example, cyprinodonts, frogs, lizards, chickens, mice, rats, guinea pigs, hamsters, rabbits, dogs, cats, sheep, pigs, goats, cow, and monkeys.

Examples

The present invention will be described in more detail with reference to Examples below. However, the present invention is not limited to these Examples by any means.

(Example 1)

(Example 1-1)

- Preparation of *in vitro* BBB model -

Cerebrovascular endothelial cells (rat brain endothelial cells: RBECs) were isolated according to Reference 1 shown below. The details are as follows:

A 3-week-old Wistar rat was anesthetized with ether and then decapitated. The cerebrum was excised and placed in a dish on ice. After removal of the meninges, the cerebral cortex was cut finely in a dish on ice. The slices were enzymatically treated by shaking (200 rpm) at 37°C for 1.5 hours with collagenase (CLS2) (1 mg/ml; Worthington Biochemical Corp.) and deoxyribonuclease I (50 units/ml; Sigma-Aldrich Corp.). After centrifugation, 20% bovine serum albumin (BSA)-DMEM was added to the obtained pellet, and the mixture was centrifuged (1000 x g, 20 minutes) to remove neurons and glia cells. Then, the residue was enzymatically treated by shaking (200 rpm) at 37°C for 30 minutes with collagenase/dispase (1 mg/ml; Boehringer Mannheim K.K.) and deoxyribonuclease I (50 units/ml; Sigma-Aldrich Corp.). After centrifugation, the obtained pellet was suspended in a small amount of DMEM and added to a 33% Percoll (GE Healthcare Japan Corp.) solution with a density gradient formed in advance at 30000 x g at 4°C for 1 hour. The mixture was centrifuged to isolate brain capillary fractions.

The brain capillary fractions thus isolated were cultured at 37°C under 5% $CO_2$/95% atmosphere in DMEM/F12 containing 10% plasma derived serum (PDS), 50 $\mu$g/mL gentamicin, 1 mM L-glutamine, 1 mg/mL heparin, 1.5 ng/mL bFGF, 5 $\mu$g/mL insulin, 5 $\mu$g/mL transferrin, 5 ng/mL selenium, and 4 $\mu$g/mL puromycin (RBEC culture solution I) using a culture dish coated with collagen and fibronectin. Forty eight hours later, the medium was replaced with RBEC culture solution I except for puromycin (RBEC culture solution II) The resultant cells were further cultured to obtain cerebrovascular endothelial cells (RBECs).

A monolayer *in vitro* BBB model, which was a monolayer RBEC culture system, was prepared from the RBECs thus obtained using Transwell (registered trademark) (24-well type, Corning Costar Corp., MA).

Specifically, Transwell insert (12-well type, Corning Costar Corp., MA) with a polycarbonate mem-

brane (0.4 μm pore size) coated with collagen and fibronectin was placed in each well of a 24-well culture plate (Corning Costar Corp., MA). RBECs ($5.0 \times 10^4$ cells/well) were inoculated in the insert (monolayer). On the next day of RBEC inoculation, the solution was replaced with RBEC culture solution II containing hydrocortisone (500 nM). Two days later, a completed *in vitro* BBB model was used in experiments.

(Example 1-2)

- Permeability experiment -

The influence of metformin on BBB functions was confirmed with the permeability coefficients of fluorescein sodium (Na-F) (Sigma-Aldrich Corp., St. Louis, MO) and Evans blue-albumin (albumin) (Evans blue; Sigma-Aldrich Corp., E2129, and bovine serum albumin; Sigma-Aldrich Corp., A7906) as an index.

In this context, a vascular relaxation factor adrenomedullin enhances blood-brain barrier functions, thereby suppressing the permeation of fluorescein sodium *in vitro* and *in vivo* (Non Patent Literature 16 and the unpublished data of the present inventor). Specifically, *in vitro* and *in vivo* tests on the permeation of fluorescein sodium have a positive relationship.

The test samples used were 0.1 mM metformin (metformin hydrochloride; Sigma-Aldrich Corp., D15,095-9), 0.5 mM metformin (the same as above), and 1 mM metformin (the same as above) each dissolved in RBEC culture solution II except for PDS. The control group used was RBEC culture solution II except for PDS. The culture solution in the *in vitro* BBB model completed as above was fully removed and instead replaced with each test sample. 24 hours later, the permeability test was conducted.

A physiological buffer (0.1 ml) containing fluorescein sodium (100 μg/ml) or albumin (0.04 g/ml) was added to the vascular side of the insert. 10, 20, 30, 60, 120, and 180 minutes later, a sample (0.4 ml) was collected from each well (brain side). A fresh physiological buffer was added thereto in the same amount as above. The fluorescence intensity of fluorescein sodium was measured (excitation wavelength: 485 nm, fluorescence wavelength: 530 nm) using a fluorescence plate reader (CytoFluor (registered trademark) Series 4000, PerSeptive Biosystems, Inc., Framingham, MA). The absorption intensity of albumin (wavelength: 630 nm) was measured using an absorption plate reader (Sunrise (registered trademark), TECAN Group Ltd., Mannedorf, Switzerland). The concentrations of fluorescein sodium and albumin were calculated from calibration curves.

Clearance and permeability coefficients (P) were calculated according to References 1 and 2 shown below. The clearance was defined as the amount (indicated by μL) of fluorescein sodium or albumin delivered from the chamber on the vascular side to the chamber on the brain parenchyma side and calculated according to the following expression from the initial concentration $[C]_L$ of fluorescein sodium or albumin placed in the vascular side and the final concentration $[C]_A$ of fluorescein sodium or albumin delivered to the brain parenchyma side:

$$\text{Clearance } (\mu l) = [C]_A \times V_A / [C]_L$$

($V_A$: Volume (1.5 ml) of the chamber on the brain parenchyma side)

The permeability coefficient P (cm/min) was determined according to the following expression:

$$1 / PS_{app} = 1 / PS_{membrane} + 1 / PS_{trans}$$

PS represents the slope of a line on which clearance was plotted against time and is indicated by (Permeability coefficient) x (Surface area of the membrane). $P_{app}$ represents an apparent permeability coefficient. $P_{trans}$ represents a true permeability coefficient. $P_{membrane}$ represents a permeability coefficient derived from only the membrane of the chamber.

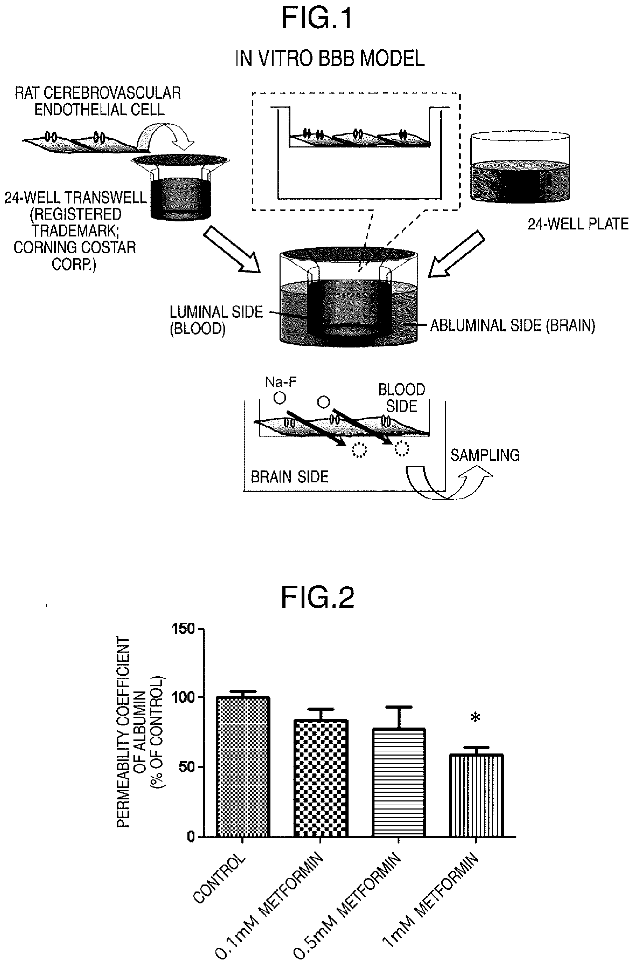

A schematic diagram of this test is shown in Fig. 1.

The test was conducted with reference to the literatures described above and the following references:

[Reference 1]
Isobe, I., Watanabe, T., Hazemoto, N., Yamagata, K., Ueki, T., Nakanishi, K., Asai, K., Kato, T., "Astrocytic contributions to blood-brain barrier (BBB) formation by endothelial cells: a possible use of aortic endothelial cell for in vitro model", Neurochemistry International, 1996, 28, p.523-533

[Reference 2]
Dehouck, M.-P., Jolliet-Riant, P., Bree, F., Fruchart J.-C., Cecchelli, R., Tillement, J.-P., "Drug transfer across the blood-brain barrier: correlation between in vitro and in vivo models", Journal of Neurochemistry, 1992, 58, p.1790-1797

[Reference 3]
Hayashi Y., Nomura M., Yamagishi S., Harada S., Yamashita J., Yamamoto H., "Induction of various blood-brain barrier properties in non-neural endothelial cells by close apposition to co-cultured astrocytes", Glia, 1997, 19, p.13-26

[Reference 4]
Takata F, Sumi N, Nishioku T, Harada E, Wakigawa T, Shuto H, Yamauchi A, Kataoka Y, "Oncostatin M induces functional and structural impairment of blood-brain barriers comprised of rat brain capillary endothelial cells", Neuroscience Letters, 2008, 441(2), p.163-166

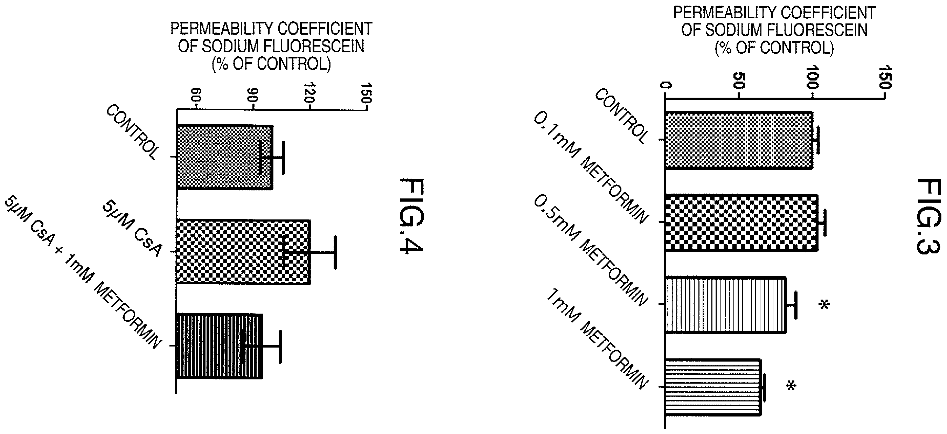

Test results obtained using each concentration (0.1 mM, 0.5 mM, and 1 mM) of metformin with test results (permeability coefficient) of the control group as 100% are shown in Figs. 2 and 3. Fig. 2 shows the results of determining the permeability coefficient of albumin. Fig. 3 shows the results of determining the permeability coefficient of fluorescein sodium.

In the test to determine the permeability coefficient of albumin, the one-way ANOVA method of 4 groups produced a significant difference with a significance probability P of 0.0009 and a significance level of 0.05. A significance test further conducted using a Dunnett's multiple comparison test method with a significance level of 0.05 between the control group and each metformin group resulted in a significant difference of the 1 mM metformin group. This group is marked with * in Fig. 2.

In the test to determine the permeability coefficient of fluorescein sodium, the one-way ANOVA method of 4 groups produced a significant difference with a significance probability P less than 0.0001 and a significance level of 0.05. A significance test further conducted using a Dunnett's multiple comparison test method with a significance level of 0.05 between the control group and each metformin group resulted in a significant difference of the 0.5 mM metformin group and the 1 mM metformin group. These groups are marked with * in Fig. 3.

As shown in Figs. 2 and 3, metformin significantly suppressed the permeability of albumin or a tight junction marker fluorescein sodium from blood side into brain side, compared with the control group.

(Example 2)

Protective effect of metformin on blood-brain barrier dysfunction induced by cyclosporin A (CsA) (central adverse reaction-inducing drug).

An immunosuppressive drug cyclosporin A (CsA) is a beneficial medicament that improves the success rate of organ transplantation. On the other hand, this drug has many adverse reactions. Its administration must be discontinued when central adverse reactions such as tremor or convulsion occur. We have previously revealed that the increased permeability of CsA into the brain in association with CsA-induced decline in blood-brain barrier functions is involved in the occurrence of central adverse reactions. Thus, metformin was examined for its effect on the CsA-induced decline in blood-brain barrier functions.

(Example 2-1)

An *in vitro* BBB model was prepared according to Example 1-1.

(Example 2-2)

A permeability experiment was conducted in the same way as in Example 1-2 except that a different method for drug stimulation was used.

The test samples used were CsA dissolved in ethanol and 1 mM metformin (metformin hydrochloride; Sigma-Aldrich Corp., D15,095-9) dissolved in RBEC culture solution II except for PDS. The control group used was ethanol and RBEC culture solution II except for PDS. The culture solution in the *in vitro* BBB model completed as above was fully removed and instead replaced with each test sample. Twenty four hours later, the permeability test was conducted.

The results of this test are shown in Fig. 4. As shown in the diagram, the loading of CsA increased the permeability of fluorescein sodium, whereas the combined use with metformin suppressed the CsA-induced decline in blood-brain barrier functions, thereby significantly suppressing the increase in the permeability of fluorescein sodium.

(Example 3)

*In vitro* effect of metformin on blood-brain barrier dysfunction induced by tissue plasminogen activator (t-PA) (central adverse reaction-inducing drug).

A "tissue plasminogen activator (t-PA)", a thrombolytic drug for cerebral infarction, exhibits very favorable effects when intravenously administered by 3 to 4.5 hours from onset. The thrombolytic therapy using t-PA, however, has disadvantages. One of the disadvantages is a therapeutic window as very short as 3 hours after cerebral infarction (Non Patent Literature 2) because blood-brain barrier dysfunction associated with use of t-PA under ischemic conditions may be complicated by hemorrhagic infarction (Non Patent Literatures 17 and 18). Thus, a "t-PA-induced BBB dysfunction model" was prepared by the loading of ischemia and t-PA onto an *in vitro* BBB model. Metformin was examined for its protective effect on blood-brain barrier functions.

(Example 3-1)

An *in vitro* BBB model was prepared according to Example 1-1.

(Example 3-2)

A permeability experiment was conducted in the same way as in Example 1-2 except that a different method for drug stimulation was used.

The test samples used were a t-PA injection (Activacin; Kyowa Hakko Kirin Co., Ltd.) and 1 mM metformin (metformin hydrochloride; Sigma-Aldrich Corp., D15,095-9) dissolved in RBEC culture solution I except for PDS. The control group used was RBEC culture solution I except for PDS. The culture solution in the *in vitro* BBB model completed as above was fully removed and instead replaced with each test sample. The resulting sample was left for 48 hours under 95% $N_2$/5% $CO_2$ (ischemia) conditions. Then, the permeability test was conducted.

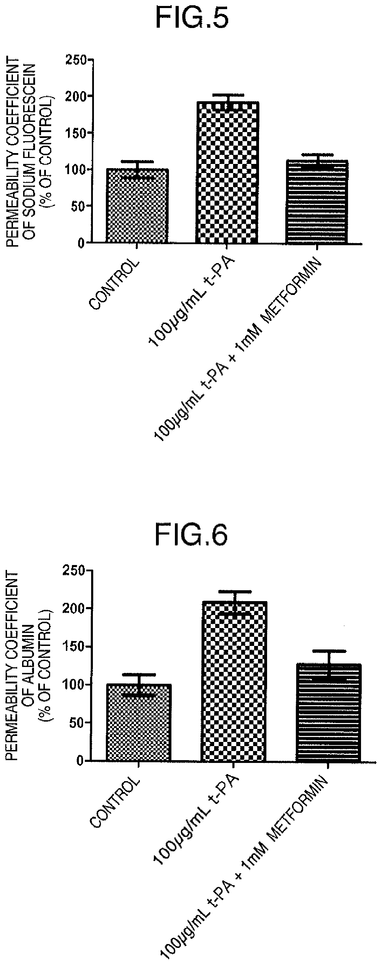

The results of this test are shown in Figs. 5 and 6. As shown in both the diagrams, the loading of t-PA under ischemic conditions increased the permeability of fluorescein sodium and albumin (Evans blue-albumin: EBA), whereas the combined use with metformin suppressed the t-PA-induced decline in blood-brain barrier functions, thereby significantly suppressed the increase in the permeability of fluorescein sodium and albumin.

(Example 4)

*In vivo* effect of metformin on blood-brain barrier dysfunction induced by t-PA (central adverse reaction-inducing drug)

(Example 4-1)

Preparation of *in vivo* t-PA-induced BBB dysfunction model

Both common carotid arteries of each ddY mouse were ligated (2VO) for 30 minutes to prepare a cerebral infarction mouse model. After reopening of blood vessels, t-PA (10 mg/kg) was administered to the subclavian vein of the resulting mouse. The mouse was left for 24 hours and used as a t-PA-induced BBB dysfunction mouse model.

Metformin was administered at a dose of 100 mg/kg concurrently with t-PA.
The control group used was a drug-unadministered cerebral infarction mouse model.

(Example 4-2)

Evaluation of BBB function

24 hours later after the administration of t-PA or the administration of t-PA and metformin, 200 $\mu$L of a mixed solution of fluorescein sodium (Na-F) (6 mg/mL) and Evans blue (EB) (20 mg/mL) was administered to the subclavian vein of the mouse. The mouse was left for 60 minutes. Then, the whole brain was excised. BBB functions were evaluated using a photograph of the whole brain and the concentrations of Na-F and EB in whole brain homogenates.

Results

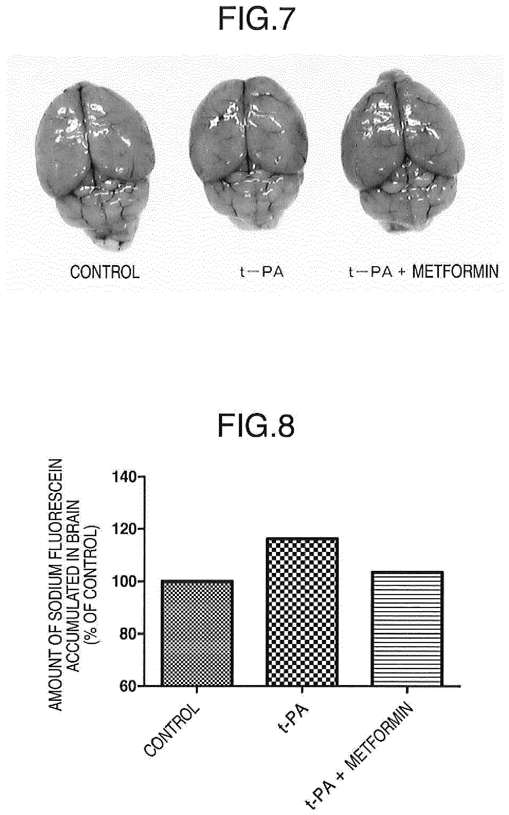

When t-PA was loaded onto the cerebral infarction mouse model, its brain was stained blue, demonstrating that EB was leaked from blood vessels into the whole brain (Fig. 7, middle). The combined use thereof with metformin lightened the blue color of the brain (Fig. 7, right), demonstrating that EB leakage from blood vessels was suppressed. This indicates that the combined use with metformin suppressed BBB dysfunction caused by the loading of t-PA.

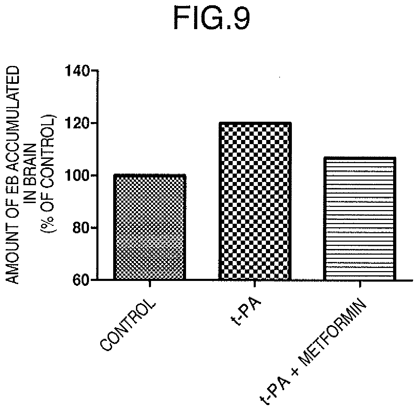

The loading of t-PA onto the cerebral infarction mouse model increased the amounts of Na-F and EB transported into the brain by 16% and 20%, respectively (Figs. 8 and 9). The combined use with metformin suppressed the increase in the transport of Na-F and EB by 78% and 66%, respectively (Figs. 8 and 9).

**Claims**

1. A medicament for use in treating blood-brain barrier dysfunction syndrome, comprising a biguanides agent which is metformin or buformin or a pharmaceutically acceptable salt thereof as an active ingredient.

2. The medicament for use according to claim 1, wherein the blood-brain barrier dysfunction syndrome is derived from a disease selected from the group consisting of diabetes mellitus, obesity, hypertension, cerebral infarction, cerebral trauma, sepsis, multiple sclerosis, Alzheimer's disease, and combination thereof, or attributed to a substance inducing the central adverse reactions.

3. The medicament for use according to claim 1, wherein the medicament is used in combination with a thrombolytic drug.

4. The medicament for use according to claim 1, wherein the medicament prevents or suppresses transport of an immunosuppressive or thrombolytic drug into the brain.

5. The medicament for use according to claim 4, wherein the medicament prevents or suppresses said drug transport into the brain, thereby preventing, suppressing, or ameliorating an adverse reaction that affects the animal or human central nervous system, preferably wherein said adverse reaction is selected from tremor, convulsion, leukoencephalopathy, headache, sleepiness, disturbance of consciousness, abnormal behavior, delirium, hallucination, delusion, epilepsy, cerebral infarction, cerebral hemorrhage and combination thereof.

6. The medicament for use according to claim 4 or 5, wherein said drug is an immunosuppressive drug.

7. The medicament for use according to claim 6, wherein the immunosuppressive drug is cyclosporine A (CsA).

8. The medicament for use according to claim 4 or 5, wherein said drug is a thrombolytic drug.

**9.** The medicament for use according to claim 8, wherein the thrombolytic drug is tissue plasminogen activator (t-PA).

**10.** The medicament for use according to any one of claims 1 to 9, wherein said biguanides agent or the pharmaceutically acceptable salt thereof is metformin hydrochloride or buformin hydrochloride.

**11.** The medicament for use according to claim 10 which said biguanides agent or the pharmaceutically acceptable salt thereof is metformin hydrochloride.

**12.** A medicament for use in treating blood-brain barrier dysfunction syndrome in combination with cyclosporin A (CsA) or tissue plasminogen activator (t-PA), comprising a biguanides agent or a pharmaceutically acceptable salt thereof as an active ingredient, and wherein said biguanides agent or the pharmaceutically acceptable salt thereof is metformin hydrochloride.

**13.** The medicament for use according to any one of claims 1 to 12, wherein a dose of the active ingredient is 125 to 3000 mg/day/person.

**Patentansprüche**

**1.** Medikament, das als Wirkstoff einen Biguanid-Wirkstoff umfasst, der Metformin oder Buformin oder ein pharmazeutisch verträgliches Salz davon ist, zur Verwendung bei der Behandlung eines Blut-Hirn-Schranken-Dysfunktionssyndroms.

**2.** Medikament zur Verwendung nach Anspruch 1, wobei das Blut-Hirn-Schranken-Dysfunktionssyndrom von einer Erkrankung stammt, die ausgewählt ist aus der Gruppe bestehend aus Diabetes mellitus, Adipositas, Bluthochdruck, Gehirninfarkt, Gehirntrauma, Sepsis, multipler Sklerose, Alzheimer und Kombinationen davon, oder auf einen Stoff zurückzuführen ist, der die zentralen Nebenwirkungen auslöst.

**3.** Medikament zur Verwendung nach Anspruch 1, wobei das Medikament in Kombination mit einem thrombolytischen Arzneistoff verwendet wird.

**4.** Medikament zur Verwendung nach Anspruch 1, wobei das Medikament den Transport eines immunsuppressiven oder thrombolytischen Arzneistoffs in das Gehirn verhindert oder unterdrückt.

**5.** Medikament zur Verwendung nach Anspruch 4, wobei das Medikament den Transport des Arzneistoffs in das Gehirn verhindert oder unterdrückt und dadurch eine Nebenwirkung verhindert, unterdrückt oder verbessert, die das tierische oder humane zentrale Nervensystem beeinträchtigt, wobei die Nebenwirkung bevorzugt ausgewählt ist aus Tremor, Konvulsion, Leukoenzephalopathie, Kopfschmerz, Müdigkeit, Bewusstseinsstörung, Verhaltensstörung, Delirium, Halluzination, Wahnvorstellung, Epilepsie, Gehirninfarkt, Gehirnblutung und Kombinationen davon.

**6.** Medikament zur Verwendung nach Anspruch 4 oder 5, wobei der Arzneistoff ein immunsuppressiver Arzneistoff ist.

**7.** Medikament zur Verwendung nach Anspruch 6, wobei der immunsuppressive Arzneistoff Cyclosporin A (CsA) ist.

**8.** Medikament zur Verwendung nach Anspruch 4 oder 5, wobei der Arzneistoff ein thrombolytischer Arzneistoff ist.

**9.** Medikament zur Verwendung nach Anspruch 8, wobei der thrombolytische Arzneistoff Gewebe-Plasminogen-Aktivator (tissue plasminogen activator; t-PA) ist.

**10.** Medikament zur Verwendung nach einem der Ansprüche 1 bis 9, wobei der Biguanid-Wirkstoff oder das pharmazeutisch verträgliche Salz davon Metformin-Hydrochlorid oder Buformin-Hydrochlorid ist.

**11.** Medikament zur Verwendung nach Anspruch 10, wobei der Biguanid-Wirkstoff oder das pharmazeutisch verträgliche Salz davon Metformin-Hydrochlorid ist.

**12.** Medikament, das als Wirkstoff einen Biguanid-Wirkstoff oder ein pharmazeutisch verträgliches Salz davon umfasst, und wobei der Biguanid-Wirkstoff oder das pharmazeutisch verträgliche Salz davon Metformin-Hydrochlorid ist, zur Verwendung bei der Behandlung eines Blut-Hirn-Schranken-Dysfunktionssyndroms in Kombination mit Cyclosporin A (CsA) oder Gewebe-Plasminogen-Aktivator (t-PA).

**13.** Medikament zur Verwendung nach einem der Ansprüche 1 bis 12, wobei eine Dosis des Wirkstoffs 125 bis 3000 mg/Tag/Person beträgt.

**Revendications**

**1.** Médicament pour utilisation dans le traitement d'un syndrome de rupture de la barrière hémato-encéphalique, comprenant, comme ingrédient actif, un agent de type biguanide qui est de la metformine ou de la buformine, ou un sel pharmacologiquement admissible de l'une de celles-ci.

2. Médicament pour utilisation conforme à la revendication 1, pour lequel le syndrome de rupture de la barrière hémato-encéphalique dérive d'une maladie choisie dans l'ensemble formé par les suivantes : diabète sucré, obésité, hypertension, infarctus cérébral, traumatisme cérébral, septicémie, sclérose en plaques, maladie d'Alzheimer, ainsi que leurs combinaisons, ou d'une maladie attribuée à une substance induisant des effets secondaires néfastes au niveau central.

3. Médicament pour utilisation conforme à la revendication 1, lequel médicament est utilisé en combinaison avec un agent thrombolytique.

4. Médicament pour utilisation conforme à la revendication 1, lequel médicament prévient ou supprime le transport d'un agent thérapeutique immunosuppresseur ou thrombolytique dans le cerveau.

5. Médicament pour utilisation conforme à la revendication 4, lequel médicament prévient ou supprime ledit transport d'agent thérapeutique dans le cerveau, et ainsi prévient, supprime ou amoindrit un effet secondaire néfaste qui affecte le système nerveux central d'un humain ou d'un animal, étant entendu que, de préférence, cet effet secondaire néfaste est choisi parmi les suivants : tremblements, convulsions, leuco-encéphalopathie, maux de tête, somnolence, troubles de la conscience, anomalies de comportement, délire, hallucinations, égarement, épilepsie, infarctus cérébral, hémorragie cérébrale, ainsi que leurs combinaisons.

6. Médicament pour utilisation conforme à la revendication 4 ou 5, pour lequel ledit agent thérapeutique est un immunosuppresseur.

7. Médicament pour utilisation conforme à la revendication 6, pour lequel ledit immunosuppresseur est la cyclosporine A (CsA).

8. Médicament pour utilisation conforme à la revendication 4 ou 5, pour lequel ledit agent thérapeutique est un thrombolytique.

9. Médicament pour utilisation conforme à la revendication 8, pour lequel ledit thrombolytique est l'activateur tissulaire du plasminogène (t-PA).

10. Médicament pour utilisation conforme à l'une des revendications 1 à 9, dans lequel ledit agent de type biguanide ou son sel pharmacologiquement admissible est du chlorhydrate de metformine ou du chlorhydrate de buformine.

11. Médicament pour utilisation conforme à la revendication 10, dans lequel ledit agent de type biguanide ou son sel pharmacologiquement admissible est du chlorhydrate de metformine.

12. Médicament pour utilisation dans le traitement d'un syndrome de rupture de la barrière hémato-encéphalique en combinaison avec de la cyclosporine A (CsA) ou de l'activateur tissulaire du plasminogène (t-PA), comprenant, comme ingrédient actif, un agent de type biguanide ou un sel pharmacologiquement admissible d'un tel agent, et dans lequel ledit agent de type biguanide ou son sel pharmacologiquement admissible est du chlorhydrate de metformine.

13. Médicament pour utilisation conforme à l'une des revendications 1 à 12, pour lequel la dose d'ingrédient actif, pour une personne, vaut de 125 à 3000 mg/jour.

# FIG.1

## IN VITRO BBB MODEL

RAT CEREBROVASCULAR
ENDOTHELIAL CELL

24-WELL TRANSWELL
(REGISTERED
TRADEMARK;
CORNING COSTAR
CORP.)

24-WELL PLATE

LUMINAL SIDE
(BLOOD)

ABLUMINAL SIDE (BRAIN)

Na-F

BLOOD
SIDE

SAMPLING

BRAIN SIDE

# FIG.2

PERMEABILITY COEFFICIENT OF ALBUMIN (% OF CONTROL)

CONTROL
0.1mM METFORMIN
0.5mM METFORMIN
1mM METFORMIN

# FIG.3

# FIG.4

# FIG.5

# FIG.6

# FIG.7

CONTROL          t−PA          t−PA + METFORMIN

# FIG.8

# FIG.9

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2009221217 A

- WO 2007124308 A

**Non-patent literature cited in the description**

- *Neuron,* 2008, vol. 57, 178-201
- *Nature Medicine,* July 2008, vol. 14 (7), 731-737
- **NICOLAS W. ; FLORENCE M. ; SYLVIE C. ; PIERRE-OLIVIER C.** The blood-brain barrier in brain hoeostasis and neurological diseases. *Biochimica et Biophysica Acta,* 2009, vol. 1778, 842-857
- **NISHIOKU T. ; YAMAUCHI A. ; TAKATA F. ; WATANABE T. ; FURUSHO K. ; SHUTO H. ; DOHGU S. ; KATAOKA Y.** Disruption of the blood-brain barrier in collagen-induced arthritic mice. *Neuroscience Letters,* 04 October 2010, vol. 482 (3), 208-211
- **YAMAUCHI A. ; DOHGU S. ; TAKATA F. ; WATANABE T. ; NISHIOKU T. ; MATSUMOTO J. ; OHKUBO Y. ; SHUTO H. ; KATAOKA Y.** Partial hepatectomy aggravates cyclosporin A-induced neurotoxicity by lowering the function of the blood-brain barrier in mice. *Life Sciences,* 14 March 2011, vol. 88 (11-12), 529-534
- **DOHGU S. ; SUMI N. ; NISHIOKU T. ; TAKATA F. ; WATANABE T. ; NAITO M. ; SHUTO H. ; YAMAUCHI A. ; KATAOKA Y.** Cyclosporin A induces hyperpermeability of the blood-brain barrier by inhibiting autocrine adrenomedullin-mediated up-regulation of endothelial barrier function. *European Journal of Pharmacology,* 10 October 2010, vol. 644 (1-3), 5-9
- **DOHGU S. ; NISHIOKU T. ; SUMI N. ; TAKATA F. ; NAKAGAWA S. ; NAITO M. ; TSURUO T. ; YAMAUCHI A. ; SHUTO H. ; KATAOKA Y.** Adverse effect of cyclosporin A on barrier functions of cerebral microvascular endothelial cells after hypoxia-reoxygenation damage in vitro. *Cellular and Molecular Neurobiology,* November 2007, vol. 27 (7), 889-899
- **TAKATA F. ; DOHGU S. ; YAMAUCHI A. ; SUMI N. ; NAKAGAWA S. ; NAITO M. ; TSURUO T. ; SHUTO H. ; KATAOKA Y.** Inhibition of transforming growth factor-beta production in brain pericytes contributes to cyclosporin A-induced dysfunction of the blood-brain barrier. *Cellular and Molecular Neurobiology,* May 2007, vol. 27 (3), 317-328

- **YAMAUCHI A. ; SHUTO H. ; DOHGU S. ; NAKANO Y ; EGAWA T. ; KATAOKA Y.** Cyclosporin A aggravates electroshock-induced convulsions in mice with a transient middle cerebral artery occlusion. *Cellular and Molecular Neurobiology,* August 2005, vol. 25 (5), 923-928
- **DOHGU S. ; YAMAUCHI A. ; NAKAGAWA S. ; TAKATA F. ; KAI M. ; EGAWA T. ; NAITO M. ; TSURUO T. ; SAWADA Y ; NIWA M.** Nitric oxide mediates cyclosporine-induced impairment of the blood-brain barrier in cocultures of mouse brain endothelial cells and rat astrocytes. *European Journal of Pharmacology,* 28 November 2004, vol. 505 (1-3), 51-59
- **YAMAUCHI A. ; OISHI R. ; KATAOKA Y.** Tacrolimus-induced neurotoxicity and nephrotoxicity is ameliorated by administration in the dark phase in rats. *Cell Mol Neurobiol,* October 2004, vol. 24 (5), 695-704
- **FUJISAKI Y ; YAMAUCHI A. ; DOHGU S. ; SUNADA K. ; YAMAGUCHI C. ; OISHI R. ; KATAOKA Y.** Cyclosporine A-increased nitric oxide production in the rat dorsal hippocampus mediates convulsions. *Life Sciences,* 20 December 2002, vol. 72 (4-5), 549-556
- **TOMINAGA K. ; KAI M. ; YAMAUCHI A. ; DOHGU S. ; TODA K. ; OISHI R. ; KATAOKA Y.** Subchronic treatment with cyclosporin A decreases the binding properties of the GABAA receptor in ovariectomized rats. *Life Sciences,* 20 December 2002, vol. 72 (4-5), 425-430
- **KANOSKI SE ; ZHANG Y ; ZHENG W. ; DAVIDSON TL.** The effects of a high-energy diet on hippocampal function and blood-brain barrier integrity in the rat. *Journal of Alzheimer's Disease,* January 2010, vol. 21 (1), 207-219
- **UENO M ; SAKAMOTO H ; TOMIMOTO H ; AKIGUCHI I ; ONODERA M ; HUANG CL ; KANENISHI K.** Blood-brain barrier is impaired in the hippocampus of young adult spontaneously hypertensive rats. *Acta Neuropathologica,* June 2004, vol. 107 (6), 532-538

- **HONDA M. ; NAKAGAWA S. ; HAYASHI K. ; KITAGAWA N. ; TSUTSUMI K. ; NAGATA I. ; NIWA M.** Adrenomedullin improves the blood-brain barrier function through the expression of claudin-5. *Cellular and Molecular Neurobiology,* 2006, vol. 26 (2), 109-118
- **JIN R. ; YANG G. ; LI G.** Molecular insights and therapeutic targets for blood-brain barrier disruption in ischemic stroke: critical role of matrix metalloproteinases and tissue-type plasminogen activator. *Neurobiol of Disease,* June 2010, vol. 38 (3), 376-385
- **ABU FANNE R. ; NASSAR T. ; YAROVOI S. ; RAYAN A. ; LAMENSDORF I. ; KARAKOVESKI M. ; VADIM P. ; JAMMAL M. ; CINES D. B. ; HIGAZI A. A.** Blood-brain barrier permeability and tPA-mediated neurotoxicity. *Neuropharmacology,* June 2010, vol. 58 (7), 972-980
- **YOKOYAMA Y. ; KUBOTA M. ; IGUCHI K. ; USUI S. ; KIHO T. ; HIRANO K.** Regulation of glyceraldehyde 3-phosphate dehydrogenase expression by metformin in HepG2 cells. *Biological & Pharmaceutical Bulletin,* 2009, vol. 32 (7), 1160-1165
- **MATSUMOTO T.** A therapeutic target for endothelium-derived hyperpolarizing factor signaling in diabetic vascular complication. *Journal of the Pharmaceutical Society of Japan,* 2010, vol. 130 (6), 777-784
- **SU E. ; FREDRIKSSON L. ; GEYER M. ; FOLESTAD E. ; CALE J. ; ANDRAE J. ; GAO Y. ; PIETRAS K. ; MANN K. ; YEPES M.** Activation of PDGF-CC by tissue plasminogen activator impairs blood-brain barrier integrity during ischemic stroke. *Nature Medicine,* 2008, vol. 14 (7), 731-737
- **SHARMA R. ; BHALLA R.** Metformin attenuates agonist-stimulated calcium transients in vascular smooth muscle cells. *Clinical and Experimental Hypertension,* 1995, vol. 17 (6), 913-929
- **LEYBAERT L. ; PAEMELEIRE K. ; STRAHONJA A. ; SANDERSON M.** Inositol-triphosphate-dependent intercellular calcium signaling in and between astrocytes and endothelial cells. *Glia,* 1998, vol. 24 (4), 398-407
- **CHARLES M. ; MORANGE P. ; ESCHWÈGE E. ; ANDRÉ P. ; VAGUE P. ; JUHAN-VAGUE I.** Effect of weight change and metformin on fibrinolysis and the von Willebrand factor in obese nondiabetic subjects: the BIGPRO1 study. Biguanides and the prevention of the risk of obesity. *Diabetes Care,* 1998, vol. 21 (11), 1967-1972
- **ISOBE, I. ; WATANABE, T. ; HAZEMOTO, N. ; YAMAGATA, K. ; UEKI, T. ; NAKANISHI, K. ; ASAI, K. ; KATO, T.** Astrocytic contributions to blood-brain barrier (BBB) formation by endothelial cells: a possible use of aortic endothelial cell for in vitro model. *Neurochemistry International,* 1996, vol. 28, 523-533
- **DEHOUCK, M.-P. ; JOLLIET-RIANT, P. ; BREE, F. ; FRUCHART J.-C. ; CECCHELLI, R. ; TILLEMENT, J.-P.** Drug transfer across the blood-brain barrier: correlation between in vitro and in vivo models. *Journal of Neurochemistry,* 1992, vol. 58, 1790-1797
- **HAYASHI Y. ; NOMURA M. ; YAMAGISHI S. ; HARADA S. ; YAMASHITA J. ; YAMAMOTO H.** Induction of various blood-brain barrier properties in non-neural endothelial cells by close apposition to co-cultured astrocytes. *Glia,* 1997, vol. 19, 13-26
- **TAKATA F ; SUMI N ; NISHIOKU T ; HARADA E ; WAKIGAWA T ; SHUTO H ; YAMAUCHI A ; KATAOKA Y.** Oncostatin M induces functional and structural impairment of blood-brain barriers comprised of rat brain capillary endothelial cells. *Neuroscience Letters,* 2008, vol. 441 (2), 163-166